(19) Europäisches Patentamt

European Patent Office

Office européen des brevets

(11) **EP 4 756 471 A1**

(12) # EUROPEAN PATENT APPLICATION

(43) Date of publication:
**10.06.2026 Bulletin 2026/24**

(21) Application number: **24217253.4**

(22) Date of filing: **03.12.2024**

(51) International Patent Classification (IPC):
**G01R 33/24** *(2006.01)*    **G01R 33/44** *(2006.01)*
**G01R 33/565** *(2006.01)*    **G01R 33/58** *(2006.01)*
**G01R 33/389** *(2006.01)*

(52) Cooperative Patent Classification (CPC):
**G01R 33/443; G01R 33/243; G01R 33/56563;**
**G01R 33/56572; G01R 33/58;** G01R 33/246;
G01R 33/389

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(71) Applicant: **Skope Magnetic Resonance
Technologies AG
8050 Zürich (CH)**

(72) Inventors:
• **Wilm, Bertram
  8003 Zürich (CH)**
• **Brunner, David
  8645 Jona (CH)**
• **Schouten, Thijmen
  3552 HA Utrecht (NL)**
• **Froidevaux, Romain
  8050 Zürich (CH)**

(74) Representative: **Hepp Wenger Ryffel AG
Friedtalweg 5
9500 Wil (CH)**

(54) **COMPUTER IMPLEMENTED METHOD, COMPUTER PROGRAM PRODUCT, COMPUTER-READABLE STORAGE MEDIUM, AND SYSTEM FOR ENHANCEMENT OF THE SIGNAL-TO-NOISE RATIO OF A MAGNETIC FIELD EVOLUTION**

(57)    The invention relates to a computer implemented method for enhancement of the signal-to-noise ratio of a magnetic field evolution for a magnetic resonance imaging apparatus (201). The method includes providing samples (S11, S21) of a magnetic field (M) acquired at a first respective point in time at at least two different spatial locations (X1, X2). The method comprises providing samples (S12', S22'; S12, S22) of the magnetic field (M) acquired at a second respective point in time at at least two different spatial locations (X1, X2). The samples (S11, S21, S12', S22'; S11, S21, S12, S22) are acquired by a field probe array (101) while the field probe array (101) is placed in the magnetic field of an imaging volume (V) of the magnetic resonance imaging apparatus (201). The method includes determining a corrected magnetic field evolution (c) with enhanced signal-to-noise ratio based on a spatial-temporal correlation of the samples (S11, S21, S12', S22'; S11, S21, S12, S22) and providing the corrected magnetic field evolution for reconstructing an image or a gradient system calibration.

EP 4 756 471 A1

Fig. 2B_1

Fig. 2B_2

Fig. 2E

**Description**

The invention relates to a computer implemented method, a computer program product, a computer-readable storage medium, and a system for enhancement of the signal-to-noise ratio of a magnetic field evolution for a magnetic resonance imaging (MRI) apparatus according to the independent claims.

An estimation/a determination of magnetic field gradients which are superimposed on a main magnetic field are essential in magnetic resonance imaging (MRI) and magnetic resonance spectroscopy (MRS).

In MRI and MRS, the spatial-temporal field evolution is determinant for image or spectrum generation. Switched gradient fields are employed for position encoding during excitation and readout as well as contrast and signal formation. The field evolution in the imaging volume of an MRI apparatus is controlled by the sequence program. The sequence program may actuate resistive coils (gradient coils and active shim coils) dynamically to control the temporal behaviour of the magnetic field. The control over the spatial encoding is exerted by actuating different coils, each with a dedicated field footprint, e.g. each gradient axis is generated by a gradient inducing resistive coil.

A magnetic resonance image reconstruction is dependent on accurate knowledge of the underlying spatial and temporal magnetic field fluctuations which may include field perturbations and eddy formations. These magnetic field fluctuations may be caused by rapid switching of gradient magnetic fields, a limited gradient bandwidth, a presence of conductive material which interacts with the magnetic field, variations in tissue properties, patient displacement/movement, imperfect shielding and/or coil design, thermal effects, and/or mechanical vibrations, e.g. of the gradient coils. These fluctuations, if unaccounted for, may adversely affect an image quality of the reconstructed images, in particular these fluctuations may lead to image artifacts, signal loss, reduced resolution, and/or a lower signal-to-noise ratio of the images.

In order to mitigate the effects of deviations for the expected field evolution, it is an established approach to measure the spatial-temporal field in an MRI apparatus and estimate a magnetic field evolution based on these measurements. The measured estimation of the field evolution inside the imaging volume is then employed to compensate or correct for the effect of the field deviations.

Dubovan, P. I., Gilbert, K. M., & Baron, C. A. (2023). A correction algorithm for improved magnetic field monitoring with distal field probes. Magnetic Resonance in Medicine; 90:2242-2260, discloses measurements of field dynamics during a scan via "field probes".

However, due to a limited number of obtainable spatially localized measurements, due to spatial constraints, and inherent noise of these localized measurements, the field estimation(s) may be subject to a low signal-to-noise ratio (SNR) which is reflected in the magnetic field evolution. In signal processing, known or hidden dimensionality restrictions and correlations can be employed for noise reduction and signal quality improvements in various ways and by various algorithms.

However, the prior art lacks a reliable, simple and effective enhancement of the signal-to-noise ratio which may enable higher resolution scans, may provide a more robust and exact method and system for reconstructing MRI images, may improve operational efficiency, and may enable operation at lower field strengths.

It is the object of the present invention to provide computer implemented methods, a computer program product, computer readable storage mediums, and a system to overcome these and further limitations and disadvantages.

One aspect of the invention relates to a computer implemented method for enhancement of the signal-to-noise ratio of a magnetic field evolution for a magnetic resonance apparatus. The method comprises providing samples of a magnetic field, in particular comprising or consisting of gradients of the magnetic field, acquired at a first respective point in time at at least two different spatial locations. The method comprises providing samples of the magnetic field acquired at a second respective point in time at at least two different spatial locations. The samples are acquired by a field probe array while the field probes are placed in the magnetic field of an imaging volume of a magnetic resonance imaging apparatus. The method further comprises determining a corrected magnetic field evolution with enhanced signal-to-noise ratio based on a spatial-temporal correlation of the samples. The method further includes providing the corrected magnetic field evolution for reconstructing an image or for calibrating a gradient system. Optionally, the method further comprises reconstructing the image or generating the gradient system calibration based on the magnetic field evolution and providing an output comprising the reconstructed image or gradient calibration.

The first point in time and the second point in time are different from each other.

The term "point in time" is to be interpreted broadly to encompass not only a precise singular moment but also a short time window. This allows for the acquisition of a sample by field probes located at different spatial positions within the short time window, rather than requiring all data to be acquired at the singular moment.

The spatial-temporal magnetic field evolution directly contains the correlations of the acquired samples in space and time, contrary to spatial-specific and/or temporal-specific correlations which are only dependent on one or the other, even if these correlations are applied sequentially. Thereby, complex spatial-temporal magnetic field evolution may be considered for noise reduction. This magnetic field evolution may enable to correct for distortions in the actuation scheme or by magnetic fields that are not induced in direct response to the sequence program such as magnetic field drifts due to heating of components, patient motion or externally generated magnetic fields.

The magnetic field evolution refers to a temporal and spatial variation of the magnetic field in the imaging volume, in particular including a static magnetic field, at least one gradient magnetic field, and/or a radiofrequency magnetic field, which are employed throughout a typical imaging procedure using the magnetic resonance imaging apparatus.

In signal processing, known or hidden dimensionality restrictions and correlations can be employed for noise reduction and signal quality improvements in various ways and by various algorithms, which may be employed in the present invention.

The magnetic field/phase evolution in the magnetic resonance imaging apparatus may not strictly adhere to the restriction in the spatial-temporal dimensionality due to actual noise on the generated fields, errors and distortions in the field generation systems, and/or emissions of external fields as well as noise and errors on the field measurement. It was found, however, that the magnetic field/phase evolution exhibits a high statistical probabilistic relation between the temporal and the spatial evolution of the field. The expected magnetic field/phase evolution in the scanner therefore has a strong bias which may be predicted by considering the spatial-temporal correlation to determine the corrected magnetic field evolution.

It was found that the dimensionality restrictions and correlations arising in the combined spatial-temporal domain are significantly larger, more influential, and more numerous than in the temporal-specific domain, in particular a spectral-specific domain, or the spatial-specific domain.

This method enables to reliably and effectively disregard spatially-temporally correlated noise when determining the corrected magnetic field evolution with an enhanced signal-to-noise ratio.

The samples may comprise or consist of measurements of k-space positions related to the magnetic field and/or measurements of phase evolutions of the magnetic field. The samples may be acquired by combining, in particular integrating, a plurality of measurements of the magnetic field over a sampling period, i.e. with a particular temporal resolution.

Alternatively or in addition, the computer implemented method may be used for enhancement of the signal-to-noise ratio of a magnetic field evolution for magnetic resonance spectroscopy or electron spin resonance (ESR) methods, in particular nuclear magnetic resonance (NMR) methods and/or electron paramagnetic resonance (EPR) methods. The present method and apparatus can be employed to enhance the measurements of field probes, e.g. magnetometers or arrays thereof, operated in MRI systems for the purpose of recording switched magnetic fields. The field probes may comprise or consist of nitrogen-vacancy (NV) center-based magnetometers, induction-based devices such as pick-up coils or vibrating/rotating coils, atomic and optical magnetometers, superconducting quantum interference devices (SQUIDs), other quantum interference devices, and/or fluxgates.

A spatial basis set can be optimized or adapted to particular systems or sequences by an appropriate choice of said basis set, e.g. by truncating combinations of various orders of spherical harmonics. The number of basis functions in the actual projection can be equal or smaller than the number of positions of the field measurement. Projecting to a lower number of spatial degrees of freedom can improve the conditioning of the inversion of P and thereby reduce noise and errors in the field estimate.

Known approaches of field monitoring previously discussed calculation of the magnetic field inside the volume under two separate assumptions. Firstly, the temporal evolution in every spatial point in the region of interest (ROI) is bandwidth-limited at a frequency substantially higher than the bandwidth of the gradient and active shim system of the MRI apparatus 201. Second, that spatial footprint is harmonic in space and of limited degree of freedom. This is employed by projecting the field evolution to a finite set of spatial basis functions and filtering the temporal field evolution (of all components) with a single filter in the temporal dimension.

However, the number of spatial-temporal degrees of freedom of the field evolution in the imaging volume V is subject to many more restrictions. In general, these degrees of freedom do not fully lie in the projection space of separated temporal filtering and spatial fitting employed in the state of the art.

The first point in time is measured in a time interval including a first dynamic comprising a plurality of samples of the field probe array and the second point is in a time interval including a second dynamic comprising a plurality of samples different from the first dynamic.

This enables to consider correlations, in particular spatial-temporal correlations, between samples of different temporally separated dynamics.

The dynamics may comprise a plurality of samples acquired over a time interval at a plurality of different spatial locations, respectively. The plurality of dynamics may be acquired at the same spatial positions at different time intervals, at different pluralities of different spatial positions at respective time intervals. Even if multiple sets of samples of a dynamic are acquired at the same time interval at different spatial locations, the samples of this dynamic may still be spatially-temporally correlated to each other. For example, a sample acquired at a first spatial location at the beginning of the dynamic may be spatially-temporally correlated with a sample acquired at a second spatial location at the end of the dynamic.

These dynamics can be substantially different from each other. One example of two dynamics being substantially different from each other may be a difference due to the magnetic resonance imaging apparatus changing its nominal gradient output, in particular in a predetermined manner, during encoding of an MRI image, e.g. due to different contrast encoding or a different k-space step. The nominal gradient output of the magnetic resonance imaging apparatus may be a nominal gradient output before applying an eddy current correction. The MRI apparatus may for example change its nominal gradient output temporally before acquisition of the dynamics, in particular at most 2 s before, in particular 20 ms before, preferably at most 2 ms before, which may still be detected during acquisition of the dynamics. Alternatively, the nominal gradient output may be changed during acquisition of the dynamic.

Another example of the dynamics being substantially different from each other can be a difference due to the magnetic resonance imaging apparatus changing its nominal gradient output after the MR system's eddy current correction stage (gradient preemphasis) during acquisition of the dynamic, in particular in a predetermined manner, for encoding of an MRI image at a distance of at most 20 cm of an MR apparatus iso-center, by at least 50 $\mu$T, in particular 1 $\mu$T, preferably 0.05 $\mu$T.

Another example of the dynamics being substantially different from each other can be that the difference of the magnetic field evolution between two dynamics at any time point of the dynamics, at any spatial point in the MR apparatus having a distance of at most 20 cm of an MR apparatus iso-center, is at least 50 $\mu$T, in particular 1 $\mu$T, preferably 0.05 $\mu$T.

Another example of the dynamics being substantially different from each other may be that the difference of a phase evolution of a proton spin related to the difference of the magnetic field evolution between two dynamics at any time point of the dynamics, at any spatial point in the MR apparatus having a distance of at most 20 cm of the MR apparatus's iso-center, is at least 1 rad, in particular 0.25 rad, preferably 0.05 rad.

Alternatively or in addition, the dynamics being substantially different from each other may be induced by drift behaviours of the MRI system, particularly its main magnet and gradient/shim field generating devices or fields induced by patient motion, breathing etc.

The dynamics being substantially different from each other may be selected from one or more of the previously mentioned examples of the dynamics being substantially different from each other.

The samples of the dynamic(s) may be acquired at a temporal resolution selected from 1 ns to 1 ms, in particular 0.5 $\mu$s to 100 us, preferably 1 $\mu$s to 10 $\mu$s.

The dynamic may be acquired at a plurality of spatial locations over a predetermined time interval. A plurality of dynamics may be acquired sequentially over sequential time intervals, optionally having the same length. The time interval for acquiring an entire dynamic may be selected from 0.1 ms to 10 s, in particular, 0.1 ms to 100 ms, preferably 0.5 ms to 80 ms.

This temporal resolution and/or this predetermined time interval may enable adequate determination of the magnetic field evolution on time scales relevant for spatial encoding and/or rapid variation of magnetic fields.

Each of the field probes of the field probe array may be configured for acquiring samples at a respective singular spatial location.

At least one of the field probes for acquiring the samples at different spatial locations may extend over a spatially extended area. A first field probe may be configured to acquire samples over a first spatially extended area. A second field probe may be configured to acquire samples over a second spatially extended area. The first and second extended areas may be different from each other. The first and second field probe may be spatially located at different spatial areas, in particular spatially overlapping spatial areas. A spatial resolution of the acquired samples at the corresponding spatial locations may be induced by methods known to encode a spatial resolution such as by switching gradient fields for phase/frequency, slice encoding, or by using spatially confined radio frequency transmission, receive fields, or combinations thereof.

The first point in time may be in a dynamic comprising a plurality of samples of the field probe array and the second point in time may be in the same dynamic.

This allows to take the correlations, especially the spatial-temporal correlation, of different samples in the same dynamic into account. Considering these different samples in the same dynamic may enable to correct the magnetic field evolution for correlated noise, in particular on small time scales.

The method may comprise providing at least a first dynamic comprising a plurality of samples and a second dynamic comprising a plurality of samples acquired over different time intervals. The method may comprise synchronizing the time intervals of acquiring the dynamics with an operation of the magnetic resonance imaging apparatus. The time intervals and the operation may be synchronized such that at least one parameter defining or contributing to a magnetic field generation is synchronized with the time intervals of acquiring the dynamic.

This enables to include correlations of entire dynamics comprising a plurality of samples to increase the signal-to-noise ratio. Thereby, more pertinent spatial-temporal correlations due to synchronization with the magnetic field generations between successively acquired samples of different dynamics may be identified.

Synchronizing the selection of time intervals to acquire the dynamics with the operation of the magnetic resonance imaging apparatus improves consistency and validity of the acquired spatial-temporal correlations with respect to a magnetic field evolution during standard operation of the MRI apparatus for image acquisition. Samples of these dynamics acquired at synchronized time intervals enable identifying these correlations while reducing variability of the samples such that noise can be distinguished in a more effective and reliable manner.

The method may include receiving a measurement initiation signal from the magnetic resonance imaging apparatus which facilitates synchronized selection of the time interval(s). The method may include sending a measurement initiation command to the magnetic resonance imaging apparatus to start the measurement which may also facilitate synchronization of the time interval(s) with the at least one parameter defining or contributing to the magnetic field. The samples may be acquired in response to the received measurement initiation signal or the sent measurement initiation command.

The operation of the magnetic resonance imaging apparatus may be a time-sequenced operation which follows a specific temporal actuation order, in particular for achieving a similar or essentially the same magnetic field in the imaging volume for the different time intervals.

The at least one parameter defining or contributing to the magnetic field generation may be a parameter indicative of a magnetic field generation of at least one gradient coil, radiofrequency coil, and/or a component of an active shimming system of the magnetic resonance imaging apparatus.

The samples and/or the dynamic(s) may have been acquired or are acquired during the method at at least 3 different spatial locations, in particular at at least 4 different spatial locations, preferably at least 16 different spatial locations.

Acquisition of samples at at least three different spatial locations enables to improve the reliability and robustness of the corrected magnetic field evolution taking into consideration magnetic field variations and corresponding correlations of magnetic field variations across the imaging volume. More spatial locations enable a more exact estimation of the magnetic field evolution, but the space in an MRI apparatus is limited such that a limited number of field probes forming a field probe array can be placed in the imaging volume. In particular, at least 16 different spatial locations enable an optimised use of space for determining the corrected magnetic field evolution while at the same time keeping the computational effort for determining the spatial-temporal correlation manageable.

Determining the corrected magnetic field evolution may comprise applying a dimensionality reduction algorithm to the samples/dynamic(s) for achieving a noise reduction, in particular (i) a principal component analysis (PCA) algorithm, singular value decomposition (SVD), or singular spectrum analysis (SSA) and/or (ii) a dimensionality reduction algorithms a locally linear embedding (LLE), a singular spectrum analysis (SSA), a Kernel-PCA, a Kernel independent PCA, a wavelet-based PCA, a wavelet-based independent PCA, isometric mapping (Isomap), t-distributed stochastic neighbour embedding (t-SNE), uniform manifold approximation and projection (UMAP), autoencoders, a machine learning, a proper orthogonal decomposition, a dynamic mode decomposition, a spatial-temporal autoencoders, a spatial-temporal convolutional neural networks, a spatial-temporal graph neural networks, a non-linear independent component analysis, a spatial-temporal variational autoencoders, and/or a spatial-temporal clustering algorithm.

Some of these dimensionality reduction algorithms enable to perform a non-linear dimensionality reduction to acquire the most pertinent spatial-temporal correlations. PCA provides an efficient linear dimensionality reduction method which is effective for modelling the magnetic field variations that can be typically modelled reliably by using harmonic functions. Kernel-PCA may further enable non-linear effects in the dimensionality reduction. LLE may enable to preserve local relationships, i.e. capture locally correlated noise. Isomap may enable to preserve the intrinsic geometry of the data. SNE, in particular t-distributed SNE, may preserve local structures of the data. UMAP enables enhanced preservation of global and local structures and is computationally fast. Autoencoders may enable to capture complex noise patterns through neural networks providing a highly customizable and powerful dimensionality reduction.

The dimensionality reduction can be applied to the dynamic(s) comprising samples of the gradient magnetic field, the integral of the gradient magnetic field, or k-space coefficients.

The dimensionality reduction by the PCA, SVD, or SSA algorithm may be chosen such as to enable a noise reduction of the corrected magnetic field evolution, in particular such that only principal components greater than or equal to a threshold are selected, in particular a threshold dependent on the samples or a predefined threshold are selected. A noise reduced field evolution may be calculated by specific weighting of singular values, in particular weighting in a predetermined manner.

This threshold, in particular predefined threshold, enables to adapt the PCA specifically to reduce spatially-temporally induced noise reliably.

The SVD may be performed using the optimal shrinkage method for singular values. The sample-dependent threshold may be determined using the Marchenko-Pastur law or the Gavish-Donoho law.

This enables to select a data dependent threshold of how many principal components to retain and filtering out components dominated by noise and, thereby, enable robust noise reduction.

For the Marchenko-Pastur method, the density of this distribution for an interval $\lambda$ in $[\lambda_{min}, \lambda_{max}]$ is:

$$\rho(\lambda) = \frac{1}{2\pi c \sigma^2} \frac{\sqrt{(\lambda_{max} - \lambda)(\lambda - \lambda_{min})}}{\lambda}, with \ \lambda_{min} = \sigma^2 (1 + \sqrt{c})^2, \qquad \lambda_{max} = \sigma^2 (1 + \sqrt{c})^2$$

The parameter $c = \dfrac{m}{n}$ denotes the ratio of the dimensions m to the number of samples n. λ denotes the eigenvalues.

For the Gavish-Donoho method, the optimal threshold formula is: $\lambda^* = \sigma\left(\sqrt{n} + \sqrt{m}\right)$ or $\lambda^* = \sigma\left(1 + \sqrt{\dfrac{m}{n}}\right)$ for large matrices and m ≤ *n*, where σ is the standard deviation, m is the number of rows, and n is the number of columns. The standard deviation $\sigma$ may be approximated by the median of the singular values of the data.

For the optimal shrinkage method, the optimal weighting method is dependent on the desired norm that is to be minimized. For example, for the case of the Frobenius norm loss, the optimal nonlinear weighting is:

$$\eta * (\sigma) = \frac{1}{\sigma}\sqrt{(\sigma^2 - \mathrm{B} - 1)^2 - 4\mathrm{B}} \ \ if \ \ \sigma \geq 1 + \sqrt{\mathrm{B}}$$

$$\eta * (\sigma) = 0 \ \ if \ \ \sigma \leq 1 + \sqrt{\mathrm{B}}$$

Parameter B denotes the ratio of the dimensions of the matrix (smallest dimension / largest dimension).

The samples may be acquired at at least two different pluralities of different spatial locations by positioning the field probe array in the magnetic field of the imaging volume at at least a first and a second position and/or orientation which are different from each other.

These first samples and the second samples acquired at different positions/orientations enable a more accurate estimation of the magnetic field by interpolation of these localized first and second samples. For example, a limited basis set of harmonic functions, such as spherical harmonic functions, based on these localized first and second samples may enable more accurate estimation of the magnetic field evolution. The order of the basis set may be equal or lower than the number of spatial locations at which the samples are acquired.

The first and second position/orientation are different from each other. The samples may be a acquired by movement/rotation of the field probe array and/or a component of the MRI apparatus within the imaging volume in a known manner, e.g. a movement along a specific distance or a rotation by a specific angular rotation.

The method can be used in context with a calibration measurement using the field probe array without a patient in the imaging volume to determine a compressed basis. The method may include receiving the compressed basis in a spatial-temporal domain, in particular in the spatial-spectral domain. The compressed basis may be derived from (i) a sub-portion measurement, (ii) a pre-scan measurement, or (iii) the calibration measurement. The method may further comprise applying the compressed basis for reconstructing an image or generating a gradient calibration.

This provides a baseline measurement which may capture MRI apparatus specific variations of the magnetic field affecting the image quality, and in particular noise. The calibration measurement without the patient may further avoid patient-induced variability. Determining a set of basis functions with the patient in the imaging volume and the compressed basis based on the calibration measurement without the patient may enable to filter between noise and artifacts which are apparatus-induced and relevant patient-induced data.

The spatial-temporal correlation may be based on measured or derived magnetic resonance imaging apparatus properties, in particular (i) a linear transfer function and/or a non-linear transfer function of the magnetic field, (ii) derived system properties of a shim system, and/or (iii) at least one nominal sequence information, selected from a nominal gradient timing, a nominal repetition time, an echo time, and/or a nominal bandwidth.

This allows to enhance the determination of the corrected magnetic field evolution based on the spatial-temporal correlation reliant on properties specific to the magnetic resonance imaging apparatus.

The computer implemented method may comprise applying a denoising kernel on temporally and/or spatially neighboring samples, e.g. the samples of the dynamic acquired over the predetermined time interval, to reduce input noise. The denoising kernel may be selected from, a Gaussian, a median, a Sobel, and a bilateral denoising kernel.

In a spherical harmonic approximation, the derived magnetic resonance imaging apparatus properties, represented by at least one set of transfer functions, may affect the harmonic components differently. For example, the transfer functions may modify the amplitude/phase of different harmonic components to a different degree.

Active shimming by the shim system may provide fine-tuning capabilities to compensate for distortions to create a more controlled magnetic field. In this manner, derived system properties of a shimming system may be considered for determining the spatial-temporal correlation.

Determining the corrected magnetic field evolution may include applying a noise reduction filter to the magnetic field evolution or to the samples/dynamic(s) in the spatial-temporal domain. The filter may be selected from a Wiener filter, a Kalman filter, or a convolutional neural network filter, and/or a Savitzky-Golay filter.

These multivariate, i.e. spatial and temporal, filters enable reliable filtering in the spatial-temporal domain such that the signal-to-noise ratio can be enhanced. Other filters may be a vector median filter, a vector directional filter, a multichannel anisotropic diffusion filter, and/or a bilateral filter for multivariate data.

The method may include applying at least one domain-specific filter, preferably a spatial domain-specific filter and/or a temporal domain-specific filter, to the magnetic field evolution or to the samples/dynamic(s).

This enables the method to selectively reduce spatial-specific correlated noise and/or temporal-specific correlated noise in addition to spatial-temporal correlated noise. Applying an additional filter, in at least one of the spatial-specific domain and/or the temporal-specific domain, provides more stable or smooth sample measurements in the spatial-specific and/or temporal-specific domain to further reduce noise.

This at least one univariate domain-specific filter may be selected from one of a Fourier transform-based regularization, in particular a low-pass filter, a high pass filter, band pass filter, and/or a wavelet filter, a Kalman filter, an averaging filter, a recursive filter, a Gaussian filter, a median filter, mean/average filter, Wiener filter, a convolutional neural network filter, and/or a Savitzky-Golay filter.

Determining the corrected magnetic field evolution or linear or non-linear spatial temporal filter based on the spatial-temporal correlation may be carried out by training a supervised or unsupervised machine learning model, in particular a neural network, preferably a convolutional neural network, optionally a residual convolutional neural network.

This machine learning model may enable an automated and adaptive optimization of noise by modifying the samples/dynamic(s) and/or the magnetic field evolution and may enable a data-driven improvement. The machine learning model may be imaging system-specific, i.e. specifically adapted for noise reduction of a specific type of magnetic resonance imaging apparatus, in particular a plurality of different types of magnetic resonance imaging apparatus. An input of the type may be received throughout the method.

Another aspect of the present invention relates to a computer program product which, when the program is executed by a computer, causes the computer to carry out the steps of the previously described computer implemented method.

Another aspect of the present invention relates to a computer-readable storage medium comprising instructions which, when executed by a computer, cause the computer to carry out the steps of the previously described computer implemented method.

Another aspect of the present invention relates to a system comprising the field probe array, in particular a magnetic resonance imaging or spectroscopy apparatus, and a processing unit configured for carrying out the previously described computer implemented method.

The field probe array may comprise an arrangement of field probes in which the field probes are arranged spaced apart at spatial locations within the imaging volume of the magnetic resonance imaging or spectroscopy apparatus. In this way, the spatial behavior of the temporal magnetic field evolution and/or spatial behavior of the time integral of the magnetic field can be estimated.

The field probe(s) may directly or indirectly (e.g. by temporal integration or differentiation) measure the magnetic field evolution, its magnitude or components thereof at a location based on various physical principles. Known principles of such magnetometers relate to but are not limited to nuclear, electron or atomic/molecular spins or momenta as in NMR/EPR or Ferro/Ferrimagnetic resonances detected inductively, in particular by using NMR/EPR field probe(s), Overhouser magnetometers, optical detectors, preferably NV center or atomic magnetometers. The field probe(s) may be functionally adapted to acquire samples based on the Hall effect, magneto resistance, induction into stationary or moving (vibrating or rotating) loops or coils, quantum interferences of particles (e.g. Aharanov-Bohm) or superconducting states (e.g. in SQUIDs), optical effects (e.g. Kerr or Faraday magnetometers), or saturation of ferro/ferri magnetic materials (e.g. fluxgates).

The field probes may be NMR or EPR field probes comprising an aqueous solution and/or at least one lipophilic component, in particular at least in proximity of means for NMR or EPR excitation and/or detection. A smallest dimension of an NMR/EPR active region of the active medium is preferably selected from one of the ranges: 0.2 mm to 0.7 mm, 0.7 mm to 1.1 mm, and 1.1 mm to 2 mm.

The invention may further enable to jointly optimize a dimension of the field probes, their arrangement in the field probe array and noise reduction. The number of field probes in the field probe array can be optimized. Also, the field probe may be selected to optimize cost, size, area, volume, resolution, and/or sensitivity for an application employing the knowledge of the effect of the denoising algorithm. Particularly, the size/area/volume of a field sensitizing entity can be reduced offering higher spatial resolution and robustness by compensating consequent loss in sensitivity or SNR by the denoising algorithm.

Furthermore, the volume of active media of NMR or EPR field probes may be reduced compared to NMR or EPR field probes operating without denoising at comparable field strength in first approximation by the factor of the SNR enhancement in the final field estimate of the denoising algorithm compared to an embodiment without denoising algorithm.

Mentioned means for NMR/EPR excitation or detection may comprise circuitry tuned for a magnetic field strength in the range selected from: 0.1 to 25 T, in particular 0.4 to 15 T.

In some embodiments, the size/volume of NMR/EPR active media is chosen to be only as large as not to impair the SNR/image quality of the MR images from particular set of applications reconstructed based on the field information derived from the field probe array and the denoising step. Impairing means that the SNR of the reconstructed images or the samples/dynamic(s) is not degraded by more than 15%, preferably 5%, in particular 1%.

NMR/EPR field probes in a typical embodiment may be specifically optimized for a magnetic field strength provided by the MRI system. This is achieved by tuning the NMR/EPR excitation and/or detection circuitry to the resonance frequency of the NMR active nucleus at said magnetic field strength of the MRI scanner.

Another aspect of the present invention relates to a computer-readable storage medium comprising the corrected magnetic field evolution with enhanced signal-to-noise ratio based on a spatial-temporal correlation obtainable by the previously described computer implemented method.

The method further enables improved differentiation between relevant signals and noise by having regard to correlations across different input data, in particular input data acquired at different nominal gradient outputs of a magnetic resonance imaging apparatus.

Another aspect of the present invention relates to a computer implemented method for enhancement of the signal-to-noise ratio of a magnetic field evolution for a magnetic resonance imaging apparatus, in particular as previously described. The method comprises providing a first dynamic comprising a plurality of first samples of the magnetic field, in particular comprising or consisting of gradients of the magnetic field, which are different from each other. The method includes providing a second dynamic comprising a plurality of second samples of the magnetic field which are different from each other. The samples are acquired by a field probe array which is placed in a magnetic field of an imaging volume of the magnetic resonance imaging apparatus. The first and second dynamic are substantially different from each other. The method comprises determining a corrected magnetic field evolution with enhanced signal-to-noise ratio based on a temporal correlation of the first and second dynamic.

This method provides a simple, reliable, efficient and time-effective computer implemented method which reliably reduces noise and improves data quality, thereby enhancing the relevant signal within the data. In particular, the method further enables to discover and take into consideration hidden noise patterns, e.g. noise anomalies and inherent noise structures within the data.

This method provides a solution to minimize noise by having regard to substantially different dynamics. Thereby, gradual shifts of samples of the dynamics, for example corresponding to the variation of the nominal gradient output, can be attributed to relevant signals while non-correlated shifts may be more easily identified as noise.

The field probe array to acquire the dynamics may comprise one or more field probes at respective spatial locations, optionally field probes at at least 2 different spatial locations, in particular at at least 3 or 4 different spatial locations, preferably at least 16 different spatial locations.

These dynamics may be acquired at disjoint time intervals, and in particular for the same spatial locations or different spatial locations.

The method may further include providing the corrected magnetic field evolution for reconstructing an image or a gradient system calibration. The method may include reconstructing the image or generating the gradient system calibration based on the corrected magnetic field evolution and providing an output comprising the reconstructed image or the gradient calibration.

The dynamics being substantially different from each other may be selected from one or more of the previously mentioned examples of the dynamics being substantially different from each other.

The samples may comprise or consist of measurements of the magnetic field magnitude, measurements of magnetic field coefficients and/or k-space coefficients related to the spatial magnetic field evolution related to spatial basis functions, in particular polynomial and higher order polynomial basis functions, measurements of the phase evolution of a spin related to the magnetic field evolution in the imaging volume (V), and/or measurements of the phase evolution of the signal of an NMR or EPR magnetic field probe. The samples may be acquired by combining, in particular by forming a temporal integral or derivative over a plurality of measurements of the magnetic field over a sampling period, i.e. with a particular temporal resolution.

The temporal correlation may be an at least partially a temporal correlation of a plurality of samples of the same dynamic.

The first and second dynamic may each respectively comprise samples acquired at a plurality of different spatial locations. The first dynamic may be acquired at at least one different spatial location than the second dynamic or the same spatial location(s) as the second dynamic.

The method may comprise synchronizing the time intervals of acquiring the dynamics with an operation of the magnetic resonance imaging apparatus. The time intervals and the operation may be synchronized such that at least one parameter defining or contributing to a magnetic field generation is synchronized with the time intervals of acquiring the dynamics.

The method may include receiving a measurement initiation signal from the magnetic resonance imaging apparatus which facilitates synchronized selection of the time interval(s). The method may include sending a measurement initiation command to the magnetic resonance imaging apparatus to start the measurement which may also facilitate synchronization of the time interval(s). The samples of the dynamics may be acquired in response to the received measurement initiation signal or the sent measurement initiation command.

The operation of the magnetic resonance imaging apparatus may be a time-sequenced operation which follows a specific temporal order. This operation may achieve a different but similar magnetic field in the imaging volume for the different time intervals.

The method may comprise providing a first dynamic acquired at a first time interval at different spatial locations. The method may comprise providing a second dynamics acquired at a second time interval at different or the same spatial locations.

The invention is now described with reference to certain embodiments and figures which show:

Figure 1: an embodiment of a system according to the invention comprising a plurality of field probes of a field probe array connected to a processing unit. The field probe array and preferably parts of the processing unit being located in or around the imaging volume of a magnetic resonance imaging or spectroscopy apparatus,

Figure 2A: a schematic illustration of a pulse sequence of switched magnetic fields produced an MRI apparatus and dynamics acquired by a field probe in the MRI apparatus,

Figure 2B: a schematic illustration of spatial-temporal correlations of samples of a dynamic acquired over a time interval,

Figure 2C: a schematic illustration of spatial-temporal correlations of samples of dynamics acquired over different time intervals,

Figure 2D: a schematic illustration of temporally correlated dynamics acquired in sequence,

Figure 2E: a schematic illustration of temporally different sequences of dynamics comprising a plurality of samples acquired at different spatial locations and the exemplary correlations between the samples and dynamics,

Figure 3: a schematic block diagram and corresponding graphs which shows a spatial-temporal magnetic field generation and determining an estimation of the spatial-temporal magnetic field, i.e. the magnetic field evolution, based on spatially localized sample measurements,

Figures 4A - 4G: exemplary graphically displayed data illustrating the effect of denoising using a SVD algorithm taking into account spatial-temporal correlations,

Figures 5A and 5B: an exemplary dataset of a root mean square error and a maximum error of the magnetic field estimation in a field of view of a field probe array acquired based on an approach according to the prior art by applying a projection and a low-pass filter,

Figures 5C and 5D: the exemplary dataset of Figs. 5A and 5B of the root mean square error and the maximum error of the magnetic field estimation in the field of view of the field probe array acquired based on a regularized inversion approach, i.e. considering the spatial-temporal correlation, and

Figures 5E and 5F: a ratio of the signal-to-noise ratios of the previously described approaches of Figs. 5C and 5D with respect to Figs. 5A and 5C as well as 5B and 5D respectively.

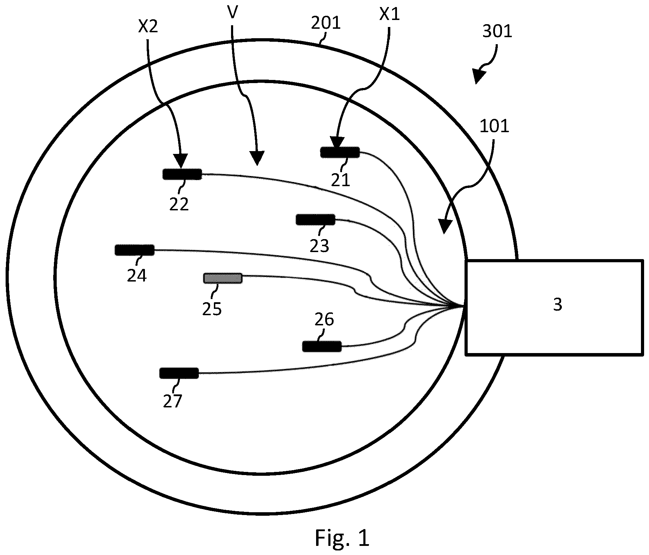

Figure 1 shows an embodiment of a system 301 according to the invention. The system 301 comprises a plurality of field probes 21, 22, 23, 24, 25, 26, 27 of a field probe array 101 which are connected to a processing unit 3. The field probes 21 - 27 of the field probe array 101 are arranged in an imaging volume V of a magnetic resonance imaging apparatus 201. The field probes 21 - 27 are arranged at different spatial location X1, X2 within the imaging volume V and configured to acquire samples of the magnetic field. The temporal resolution, i.e. sampling rate is 10 us. A dynamic comprising a plurality of samples 21 - 27 for each spatial location X1, X2 is collected, for example over a predetermined time interval of 80 ms. The operation of the magnetic resonance imaging apparatus is preferably synchronized with the acquisition time intervals of the dynamics. For example, each field probe 21 - 27 may acquire a set of samples at its spatial location X1, X2 throughout the time interval while the magnetic resonance imaging apparatus is operated in a specific temporal order, e.g. a specific spatial encoding operation.

The field in the imaging volume V can be estimated by using the field probes 21 - 27 which are formed by magnetometers arranged around or in the imaging volume V. Although these field probes 21 - 27 measure the temporal evolution of the field only in a finite set of discrete spatial locations X1, X2, the field inside the imaging volume V can be estimated based on well-established means of spatial interpolation. These spatial interpolations employ the fact that the field distribution in the quasi-stationary regime of magnetism must be represented by a harmonic function in a spatial regime. In addition, there is a limited degree of freedom of the spatial field distribution of the dynamic magnetic field prevalent in the imaging volume V.

Mathematically, this spatial interpolation is carried out by projecting the field measurements acquired at discrete locations to a limited basis set of harmonic functions ($\psi_n(\vec{r})$, e.g. spherical harmonics:

$$B(\vec{r}, t) = \sum_{n=0}^{N} a_n(t) \cdot \psi_n(\vec{r})$$

$a_n(t)$ relates to a temporal evolution of a field distribution $\psi_n(\vec{r})$ which is defined as:

$$a_n(t) = \sum_p (P^+)_{n,p} B_{r_p}(t)$$

or $a(t) = P^+ B_r(t)$ in vectorized form with $(a(t))_n = a_n(t)$ and $(B(t))_p = B_{r_p}(t)$. $P = \psi_n(r_p)$ encodes the positions of the field probes, "+" denotes an appropriate (pseudo-) inverse and $B_{r_p}(t)$ denotes the temporal magnetic field evolutions measured by the field probe located in $r_p$.

It has to be noted that the spatial basis functions $\psi_n(\vec{r})$ denoted here as being harmonic are strictly speaking only optimal in the case if the evolution of the magnetic field is calculated. In practice however, this basis set may be referred to the phase evolution of the signal phase of the spins. The magnetic field and phase relate to each other by integration in the time domain. The precession frequency of NMR and EPR scales linearly with the magnetic field strength, i.e. the norm of the magnetic field vector at the location of the spin. Therefore, the phase accrual caused by this precession in reaction to the superposition of (vector) magnetic fields can be non-linear with respect to the field superposition. Consequently, the spatial distribution this phase accrual can be of non-harmonic nature. Spatial basis functions reflecting this effect typically termed concomitant field effect may be added or used in the spatial expansion in order to improve the spatial interpolation of the phase distribution in the imaging/spectroscopy volume.

Hence, the term "spatial-temporal" degrees of freedom means degrees of freedom which are not describable in only the temporal domain, nor describable only in the spatial domain, i.e. a filter substantially projecting to such degrees of freedom is not separable to the spatial domain and the temporal domain.

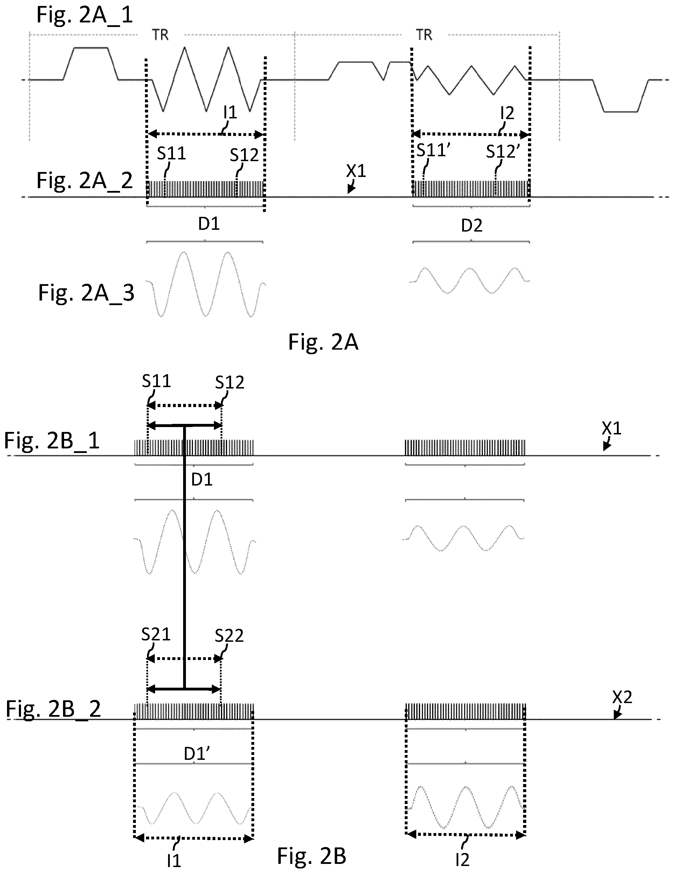

Figure 2A shows a schematic illustration of a sequence of (in general) spatially non-linear magnetic fields of an MRI apparatus and dynamics D1, D2 comprising samples S11, S12, S11', S12' acquired by a field probe in the MRI apparatus. The sequence blocks comprising of a plurality of samples S11, S12 and S11', S12' which are acquired at a constant sampling rate of 10 us over a time interval of 80 ms denote one dynamic D1, D2, respectively.

Restriction of the field evolution in an MRI apparatus 201 (see Fig. 1) to spatial-temporal degrees of freedom are induced by various mechanisms. The sequence program requests in most cases a temporally and spatially highly structured field evolution. One restriction may be that MRI and magnetic resonance spectroscopy (MRS) sequences are often times highly repetitive or nearly repetitive.

An upper subsection Fig. 2A_1 of Fig. 2A shows non-uniform magnetic fields generated by an MRI system over a prolonged period of time. The sequence of Fig. 2A employs a sequence of magnetic field actuation having a repetition time TR. Magnetic field actuations are basically repeated for each repetition time TR, i.e. result in a highly-correlated/similar non-uniform magnetic field actuation for acquiring k-space lines of the image, different slices, averages, or a series of images. The non-uniform magnetic field may have distinct patterns (e.g. polygonal shapes, in particular trapezoids and/or sinusoids). This behavior can be expressed for instance in terms of auto- and cross-covariance (correlation) functions. The repetition times TR of Fig. 2A_1 have the same length and are synchronized with parameters governing an actuation of the MRI apparatus which define the non-uniform magnetic fields generated by the MRI apparatus. Hence, both non-uniform magnetic fields of these repetition times TR of Fig. 2A_1 are similar to each other. These non-uniform magnetic fields of these repetition times TR may only differ by addition or scaling of basic waveforms, such as for example polygonal waveforms, in particular trapezoid and/or sinusoid waveforms.

A center subsection Fig. 2A_2 of Fig. 2A shows the acquired dynamics D1, D2 which each include a plurality of samples S11, S12, S11', S12' which were for example acquired at a constant sampling rate of 10 us over an acquisition time interval of 80 ms. The dynamics D1, D2 were acquired at time intervals I1, I2 synchronized with an electromagnetic actuation of the MRI apparatus, i.e. the time intervals I1, I2 relate to respective subsections or the entirety of the repetition time interval TR which includes similar or the same electromagnetic actuation to achieve the non-uniform magnetic field.

Figure 2A exemplary shows an acquisition of dynamics D1, D2 via the field probe at a single spatial location X1.

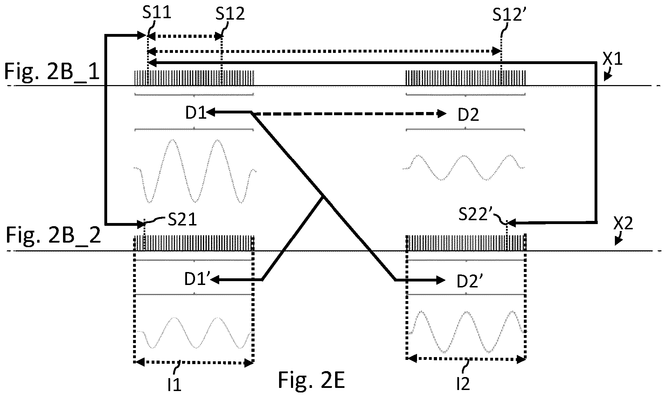

However, similarly as depicted in Fig. 2E, a dynamic D1, D2/samples S11, S12, S12', S21, S22' may be acquired at a plurality of spatial locations in addition to different time intervals I1, I2.

The lower subsection Fig. 2A_3 of Fig. 2A shows an estimation of the non-uniform magnetic field measured based on the respective dynamics D1, D2 using the field probe array. This estimation of the non-uniform magnetic field approximates the actual non-uniform magnetic field for the respective time interval I1, I2. Based on these estimations of the non-uniform magnetic field, the magnetic field evolution can be determined.

Since MRI apparatus are stable to a certain degree, the previously mentioned relations, i.e. a parameter/parameters defining or contributing to a magnetic field generation, can be expected to translate into the actual field evolution and thereby to be reflected in the magnetic field measurements. For instance, the fact that the requested field evolution is similar in every time interval I1, I2 must be reflected in the data and corrected magnetic field evolution.

A field actuation of the MRI apparatus (gradient chain and shim chain comprising of numerical waveform generation, eddy current compensation, digital-to-analogue-conversion, amplification, filtering, and gradient coil actuation) limits the degree of freedom of the spatial-temporal magnetic field evolution that can be achieved during magnetic field generation.

MRI apparatus have a limited bandwidth (typically in the range of several 10 kHz) which restricts the degrees of freedom in the temporal domain. This can be employed for denoising samples of magnetic field measurements by means of low-pass-filtering the acquired temporal magnetic field data with a cut-off frequency close to the bandwidth limitation of the magnetic field actuation devices. However, the induced limitation of degrees of freedom by an electromagnetic actuation system of the MRI apparatus are further reaching as exemplified by the higher order transfer functions of each actuation channel. These transfer functions imply that the actuation of each channel at each frequency only significantly produces one distinct spatial-spectral (and thereby spatial-temporal) field output response. The magnetic field measured by the field probe array in the MRI apparatus is therefore expected to closely follow a spatial-temporal manifold prescribed by the transfer function of each electromagnetic actuation system.

However, there are magnetic field evolutions in an MRI apparatus that are not induced by the sequence program or the actuation hardware but are generated by heating transients of involved components, subject movement or are generated by external source such as power lines, electric motors, or other MRI apparatus components. However, these emissions typically have well known power spectra, resulting in correlated shifts of the samples of magnetic field measurements. For example, the temperature and other magnet drifts are known to be very slow compared to the actuation of the gradient fields. Also, subject movements are typically limited to a few 10 Hz and may have very distinct peaks in their spectrum relating to heartbeat and breathing.

Figure 2B shows a schematic illustration of spatial-temporal correlations of samples S11, S12, S21, S22 of a dynamic D1, D1' acquired over a time interval I1. This correlation is indicated by the black arrows connecting the samples S11, S12, S21, S22 of the subsections Figs. 2B_1 and 2B_2 of Fig. 2B. The dynamic D1, D1' is acquired for two different spatial positions X1, X2.

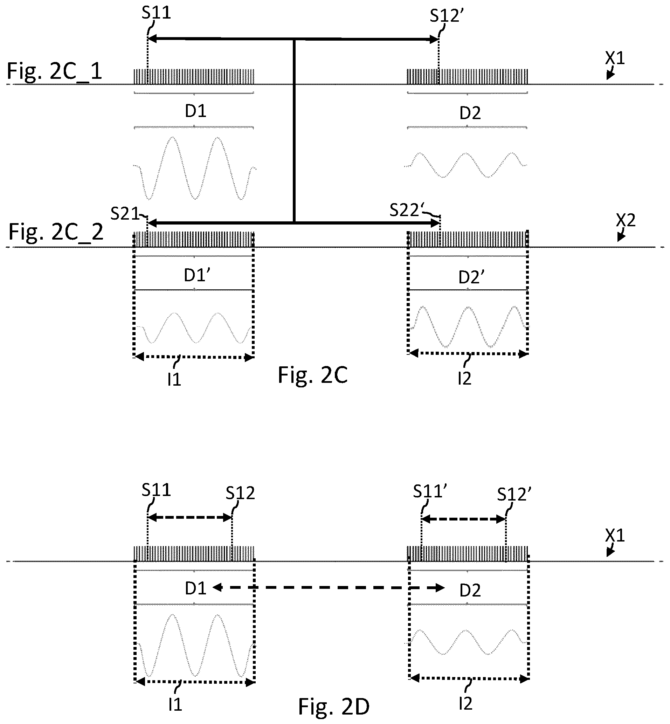

Figure 2C shows a schematic illustration of spatial-temporal correlations of samples S11, S12', S21, S22' of different dynamics D1, D1'; D2, D2' acquired over different time intervals I1, I2. This sequence of dynamics D1, D1'; D2, D2' was acquired for separate spatial locations X1, X2. The spatial-temporal correlations are exemplary shown by the black arrows connecting these samples S11, S12', S21, S22' of subsections Figs. 2C_1 and 2C_2 of Fig. 2C.

Figure 2D shows a schematic illustration of temporally correlated dynamics D1, D2 acquired in sequence over different time intervals I1, I2 which both comprise a plurality of samples S11, S12; S11', S12'. The sequence of dynamics D1, D2 of Fig. 2D is acquired at only one spatial location X1. The temporal correlation is exemplary shown as a dashed arrow connecting the two dynamics D1, D2. In addition, the temporal correlation may also cover temporally correlated samples of the same dynamic D1; D2 as indicated by the smaller dashed arrows.

Figure 2E shows a schematic illustration of temporally different sequences of two dynamics D1, D1'; D2, D2' comprising a plurality of samples S11, S12, S12', S21, S22' which are acquired at different spatial locations X1, X2 by the field probe array. For better visibility, only one sample S21 of the first dynamic D1, D1' is depicted in the acquired samples at a second spatial position X2 and only one sample S12', S22' of the second dynamic D2, D2' is depicted respectively at the first and second spatial position X1, X2. However, both dynamics D1, D1'; D2, D2' also comprise a plurality of samples S11, S12, S21, S12', S22' of different spatial locations X1, X2, respectively.

Each dynamic D1, D1'; D2, D2' was acquired over a time interval I1, I2, in particular in a synchronized manner with the electromagnetic actuation of the MRI apparatus as described in Fig. 2A. Fig. 2E further shows exemplary correlations between the samples S11, S12, S12', S21, S22' and dynamics D1, D1'; D2, D2'. The spatial-specific and temporal-specific correlations are denoted by dashed arrows between the samples S11, S12, S12', S21, S22' and dynamics D1, D1'; D2, D2'. The spatial-temporal correlations, i.e. having a temporal and spatial degree of freedom, are indicated by solid line arrows between the samples S11, S12, S12', S21, S22' and dynamics D1, D1'; D2, D2'.

Even sets of samples of a dynamic D1, D1' which were acquired at different spatial locations X1, X2 but over the same time interval I1 may be subject of a temporal correlation because individual samples S11, S12, S21, of this dynamic D1, D1' may be correlated in a temporal domain.

Figure 2E further shows, similar to Fig. 2A_3, non-uniform field estimations based on the dynamics D1, D1'; D2, D2'. Due to different spatial locations X1, X2 of field probes of the field probe array, the field estimations may differ to a degree, e.g. due to being spatially spaced apart or differently affected by noise. However, the magnetic field at the different spatial locations X1, X2 is still highly correlated in a spatial degree of freedom as well as a temporal degree of freedom. Therefore, a temporal-specific, a spatial-specific, and/or a spatial-temporal correlation may considerably contribute to measured noise and is to be minimized. For example, spatial-temporal correlated noise such as a localized magnetic field distortion, e.g. induced by coil heating or patient movement, which moves spatially as time passes can only be adequately accounted for by considering a spatial-temporal correlation. Even though Fig. 2E merely shows two different spatial locations X1, X2, these dynamics D1, D1'; D2, D2' are preferably acquired at a plurality of different spatial locations X1, X2, in particular more than 10, as schematically illustrated in Fig. 1.

The spatial-temporal field evolution in an MRI system is expected to be of significantly lower dimensionality than the typical projection approach for field estimation. Also, a strong correlation between the temporal and spatial behavior is expected to be induced by the sequence and the actuation as well as by the external sources.

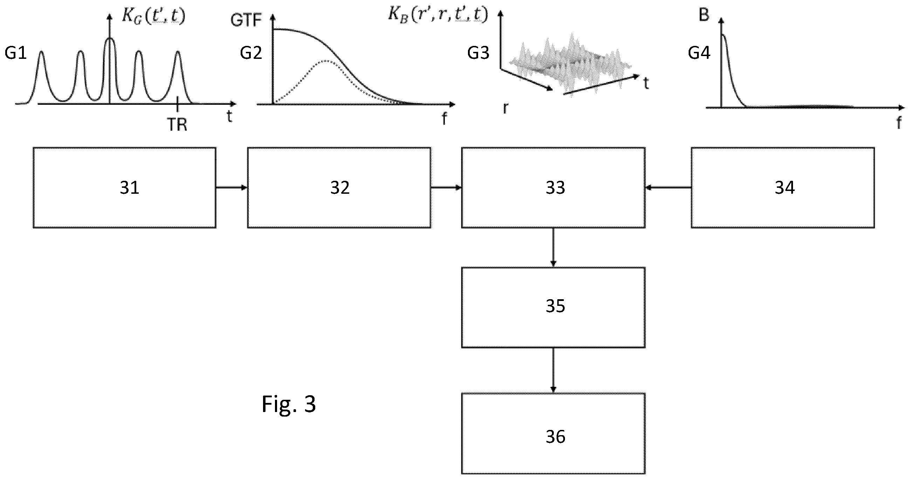

Figure 3 shows a schematic block diagram and corresponding graphs which show a spatial-temporal magnetic field generation and estimations based on spatially localized acquired measurements.

Figure 3 illustrates a spatially and temporally dependent field evolution in step 33 which is influenced by a sequence of step 31, a field actuation of step 32, and field drifts/external fields of step 34.

The graph G1 of Fig. 3 shows a magnetic field $K_G(t',t)$ of the sequence step 31 in the temporal domain t including the repetition time TR.

The graph G2 of Fig. 3 shows a generalized transfer function GTF of the field actuation of step 32 over a frequency f domain.

The graph G3 of Fig. 3 shows a cross-correlation of step 33 in the spatial and temporal domain as a three-dimensional graph $K_B(r',r,t',t)$.

The graph G4 shows the magnetic field $B(f)$ due to field drifts/external fields of step 34, i.e. noise in the frequency f domain. The field evolution 33 is locally measured by a plurality of field probes 21 - 27 (see Fig. 1) such that a plurality of local field measurements $B_{rp}(t)$ for $p = 1, ... ,n$ can be acquired in step 35. Based on these local field measurements $B_{rp}(t)$ a spatially-temporally correlated field estimation $\hat{B}(r,t,B_{rp}(t))$ can be determined in step 36, e.g. via estimation using a limited basis set of spherical harmonic functions.

By including previously described spatial-temporal correlations of different samples/dynamic(s) and/or correlations between different dynamics, the noise when estimating the field evolution 36 can be considerably reduced.

Signal correlations for field estimation denoising can be known by calibrating, calculating, or estimating information on the sequence or the field actuation device. A well-established method for measuring properties of the field actuation system is by means of measuring its transfer or impulse response function. Similar approaches can be employed to estimate a transfer function during the actual measurement. Information on the sequence can be gathered from MRI apparatus's control unit or its outputs into the actuation chain.

It has however to be noted, that for most denoising approaches presented below not the actual response function or pulse waveform is needed, but only a statistical expression of its relations. For sufficiently linear systems, these relations can be expressed by the cross-correlation/covariance functions of the output values. Information on the absolute timing is therefore not necessary, only the relative timing between the output values is of relevance.

In cases where the signal correlations cannot be known as described above, they can be implicitly estimated or employed by dimensionality reducing denoising algorithms. A standard approach is well-known in literature is based on singular value decomposition (SVD), or principal component analysis (PCA). Also, more involved variants like Singular Spectral Analysis (SSA) or sparse singular value decompositions approaches are able to employ the implicit redundancy for noise suppression and increasing data quality.

Although referring to the target estimate to be a (magnetic) field value, many applications, such as employing the field measurement in reconstruction, employ the time integral of the field. Employing NMR/EPR field probes, this entity directly relates to the phase evolution φ(t) of the RF signal emitted by the field probe:

$$\varphi(t) = \gamma \int ||B(t)|| dt.$$

However, due to the approximately linear relation between the two entities, the phase and field signal can be analogously denoised and often the algorithm can be applied to both input values. However, it may be beneficial to explicitly incorporate the temporal/spectral noise statistics of the employed field probe for the optimal estimation. In the case of NMR field probes, operating on a coherence (e.g. FID), the RF signal is subject to white Gaussian noise (thermal noise limit case). However, if field values are derived, the noise statistics of the resulting field estimate is spectrally weighted by the differentiation by a factor of ω. In case a field probe for measuring the instantaneous magnetic field or a component thereof is employed (e.g.

Hall sensor, atomic magnetometers, EPR, nitrogen vacancy (NV) center-based magnetometers etc.) and the phase signal (e.g. NMR based field probes) is integrated this results in noise weighting of $\frac{1}{\omega}$ .

**PCA/SVD Approach:**

In this embodiment, an SVD or PCA is applied to the measurements of the field probe array. The temporal data from the field probes is temporally partitioned such as to account for the temporal long-term correlations in the autocorrelation function. In a very typical case this would imply, that the data is arranged in equal time intervals relative to the repetition time TR of the sequence:

$$B_{r_p\,j}(t_\tau) = B_{r_p}(t_\tau + j \cdot TR), \qquad j = 1, \ldots, T$$

whereas $t_\tau = t - j \cdot TR$ is the relative time within the repetition time interval and j enumerates the repetitions.

In the simplest implementation, the data acquired from all field probes and repetition times are stacked in a matrix:

$$S = \begin{pmatrix} X_{r_1\,1}(t_1) & X_{r_1\,1}(t_2) & \ldots \\ X_{r_2\,1}(t_1) & X_{r_2\,1}(t_2) & \ldots \\ \ldots & \ldots & \ldots \\ X_{r_n\,1}(t_1) & X_{r_n\,1}(t_2) & \ldots \\ X_{r_1\,2}(t_1) & X_{r_1\,2}(t_2) & \ldots \\ \ldots & \ldots & \ldots \\ X_{r_n\,T}(t_1) & X_{r_n\,T}(t_2) & \ldots \end{pmatrix}$$

Note that the values X can be the magnetic field B, or its integral as phase, or k-space coefficients or other projected field or phase evolutions in a given spatial basis set.

It has to be however noted, that the data may also be analogously arranged in more than 2 dimensions, accounting for more than one potential correlation in the time domain.

The matrix S can then be decomposed using an SVD:

$$S = U\Sigma V^*$$

In the case of S having a higher dimensionality, corresponding higher-order singular value decompositions can be applied.

Due to the induced correlations and sparsity as discussed above, the singular values will have a strong decay property. Singular values below a certain threshold T can hence be nulled or filtered to denoise the data:

$$\left(P_T(\Sigma)\right)_{q,q} = \begin{cases} \Sigma_{q,q} & for\ \Sigma_{q,q} > T \\ 0\ else \end{cases}.$$

The denoised data can then be read from the back transformed matrix:

$$\hat{S} = U P_T(\Sigma) V^*.$$

As mentioned, the approach would be analogously applicable to the projected field evolution $a_n(t)$ in a given spatial basis set.

The difference between the two approaches above would be the weighting of the coefficients. The signal weighting can be introduced by well-established means. Of particular interest is weighting, that reflects the signal and noise propagation of the field interpolation into the volume/region of interest for imaging.

In a particular implementation, this can be employed by considering the signal in the ROI for compression:

$$(S_{ROI})_{c,\tau} = \sum P^+ B_{r_p}(t) \cdot \Psi_n(r_c)$$

Where $r_c$ are a set of control points within the ROI.

It is understood that estimates of the noise statistic among the field probes can be similarly incorporated in the estimate, similarly as discussed below.

As mentioned above, such an approach takes correlations in the spatial-temporal domain into account. In the prior art, often times temporal correlations are calculated for each spatial term separately, neglecting correlations between the spatial terms. Other prior art includes employing an SVD/PCA and only using the spatial compression, i.e. restricting their basis set for field interpolation and reconstruction to a basis set given by a PCA truncation.

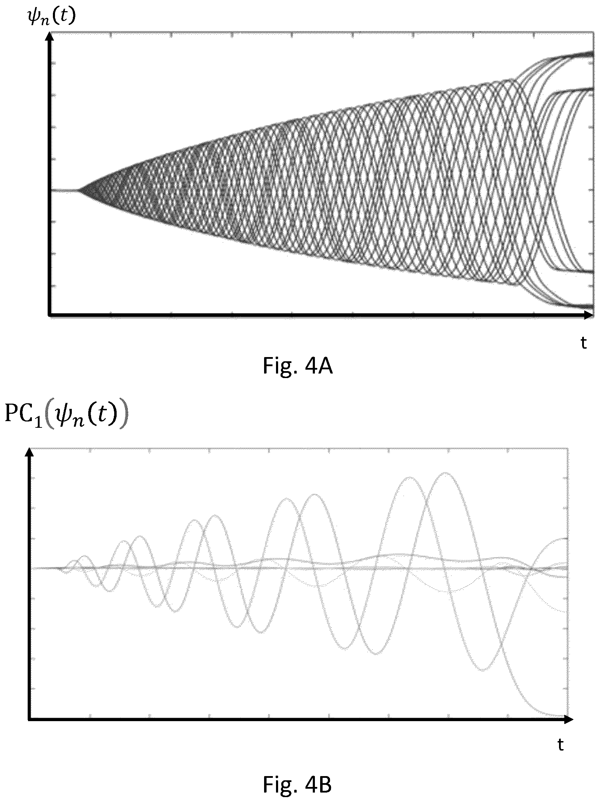

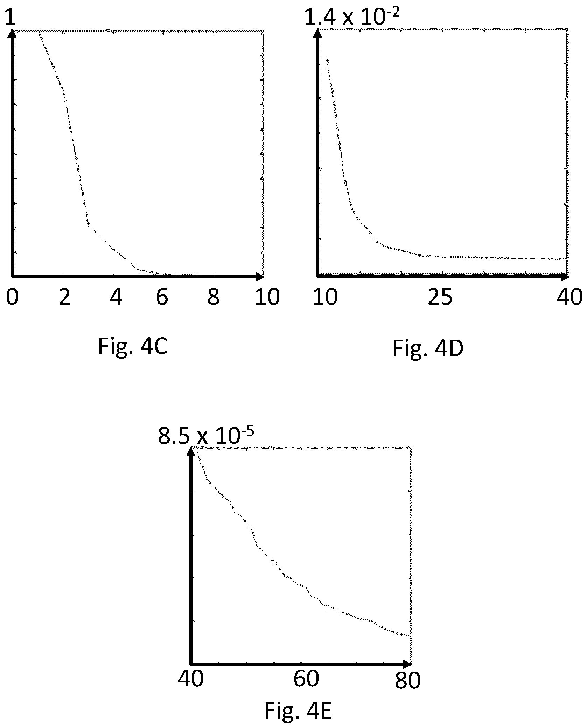

Figures 4A - 4G show exemplary graphically displayed data illustrating the effect of denoising using a PCA algorithm considering spatial-temporal correlations.

Figure 4A shows the phase $\psi_n(t)$ corresponding to the magnetic phase evolution $\psi_n(t)$ of a plurality of field probes of the field probe array comprising several substantially different dynamics of a multi-shot spiral sequence.

Figure 4B shows main singular vectors $PC_1^{(\psi_n(t))}$ showing the dominant singular values extracted from the phase corresponding to the magnetic field evolution of Fig. 4A.

Figures 4C - 4E show the singular values of 1 - 80 singular vectors in descending order. The singular values decrease rapidly, indicating that few singular vectors may be used to adequately describe the data.

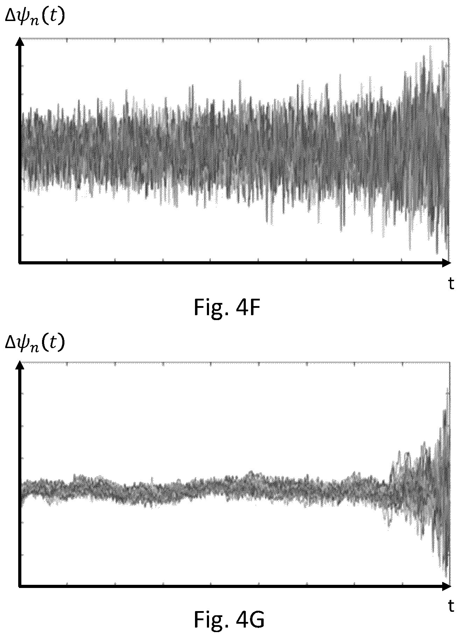

Figure 4F shows a phase evolution noise $\Delta\psi_n(t)$ before nominal gradient encoding starts with only temporal filtering being applied. For better visualizing the phase evolution noise, the phase was subtracted in Fig. 4F.

Figure 4G shows the same phase evolution noise $\Delta\psi_n(t)$ as Fig. 4G but with SVD-based denoising being applied.

**Singular Spectrum Analysis**

The approach of using singular spectrum analysis (SSA) or multi-variate/multi-channel versions is procedurally similar to the SVD approach discussed above. It is understood that the window width employed can be chosen based on the expected temporal correlations induced by the sequence and/or the system's transfer function. Denoising is achieved also here by truncation of the SVD. While direct application of the SVD to the measured field/phase evolutions, the singular spectrum analysis can capture structures within the data without denoting the repetition time explicitly. In such cases, the spectral signal analysis (SSA) is expected to provide a stronger noise reduction for spatial-temporal filtering and in principle even a certain ability of signal extrapolation as reported in literature in different applications. However, the main drawback is its higher numerical complexity. The approach of denoising via a SSA is only representative for an increasingly large set of auto-regressive methods.

**Regularized projection approach**

In another embodiment, the result of the field estimation is regularized towards a known/imposed, multi-variate statistics of the field evolution. To simplify notation, the following will be treated in Fourier/spectral domain. The filtering could be converted to time domain or finite impulse response representations.

The field evolution can be expressed in a field expansion as previously suggested. The probable field evolution terms $a_n(\omega)$ are then, assuming a transfer function $H_{nj}(\omega)$ for field actuation of channel j to basis function $\Psi_n(r)$ and a (unknown) pulse sequence $g_j(\omega)$ for channel j:

$$a_n(\omega) = \sum H_{nj}(\omega)g_j(\omega) \,.$$

The expected cross spectral power is hence:

$$a_n(\omega) \cdot a_{n'}(\omega)^* = \left(K^a(\omega)\right)_{n,n'} = \sum_{j,j'} H_{n,j}(\omega)g_j(\omega)\left(H_{n',j'}(\omega)g_{j'}(\omega)\right)^* = H(\omega)K^g(\omega)H(\omega)^*,$$

where $(K^g(\omega))_{j,j'} = g_j(\omega) \cdot g_j(\omega)^*$ is the spectral cross covariance of the sequence.

If the sequence is not known, making no assumption about the sequence would refer to choosing $(K^g(\omega))_{j,j'} = \delta_{j,j'}$. Even without explicit knowledge of the detailed pulse waveforms and their timing, $K^g(\omega)$ may be estimated more accurately in various ways such as based on the sequence repetition times or read-out properties.

$K^a(\omega)$ can then be calculated using a gradient transfer function as denoted above. The transfer function can be measured using well established methods or calculated from system properties. However, $K^a(\omega)$ can also be determined from the measurement itself, since it represents the power in the field evolution itself.

The field estimation can then be gained from solving a minimization problem of the following type:

$$argmin_{a(\omega)}\left(\|Pa(\omega) - B_r(\omega)\|_Q^2 + \lambda \cdot a^* (K^a)^+ a \right).$$

Whereas + denotes again an appropriate inverse, the norm Q-norm of a matrix X is $\|X\|_Q = X^H Q X$ and Q denotes an error weighting as for instance induced by the noise covariance of the field measurement $B_r(\omega)$ or other (multi-variate) measures of the reliability of the probe data. The latter expresses that the error should be weighted and decorrelated according to its noise level. The regularization parameter $\lambda$ can be chosen by well known criteria and techniques such as L-curve optimization, discrepancy regularization, and others. This minimization problem can be solved by well-known matrix inversions.

The resulting frequency domain multi-variate filter can then be applied to Fourier transformed measurement values or transposed into time domain representation. Appropriate truncation or refitting to a finite impulse response can further enhance numerical efficiency of its application.

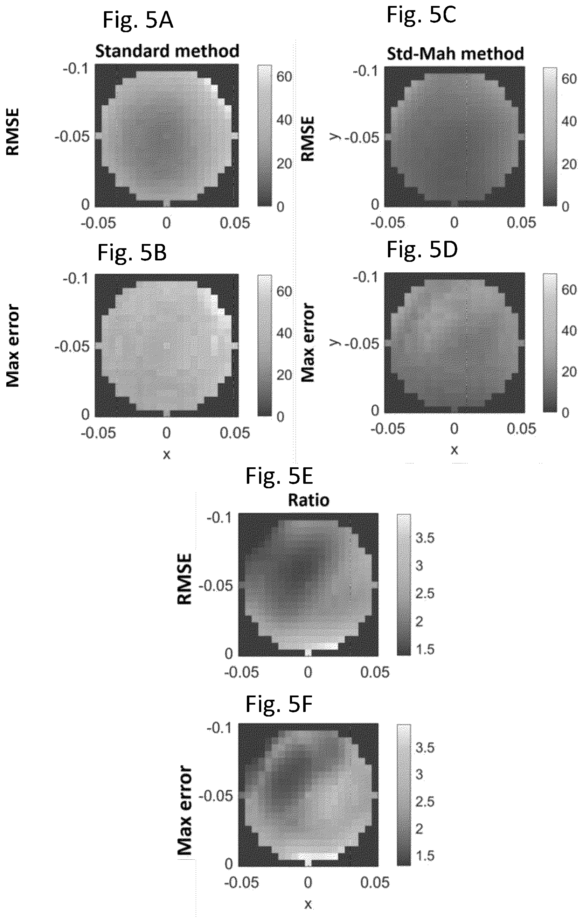

Figures 5A and 5B show an exemplary dataset of a root mean square error "RMSE" and a maximum error "Max error" of a magnetic field estimation in a field of view of a field probe array acquired based on an approach known by the prior art by applying a projection and a low-pass filter denoted as "standard method".

Figures 5C and 5D show the exemplary dataset of Figs. 5A and 5B of the root mean square error "RMSE" and the maximum error "Max error" of a magnetic field estimation in the field of view of the field probe array acquired based on a regularized inversion approach, i.e. considering the spatial-temporal correlation previously described, denoted as "Std-Mah method".

These Figs. 5A - 5D show that the "Std-Mah method" enables a more effective noise reduction for both the "RMSE" and the "Max error".

Figures 5E and 5F show a relative variation of the signal-to-noise "ratio" of the previously described approaches of Figs. 5C and 5D with respect to Figs. 5A and 5C.

**Claims**

1. Computer implemented method for enhancement of the signal-to-noise ratio of a magnetic field evolution for a magnetic resonance imaging apparatus (201) comprising the steps of:

   - Providing samples (S11, S21) of a magnetic field (M), in particular comprising or consisting of gradients of the magnetic field (M), acquired at a first respective point in time at at least two different spatial locations (X1, X2),
   - Providing samples (S12', S22'; S12, S22) of the magnetic field (M) acquired at a second respective point in time at at least two different spatial locations (X1, X2), said samples (S11, S21, S12', S22'; S11, S21, S12, S22) being acquired by a field probe array (101) while the field probe array (101) is placed in the magnetic field of an imaging volume (V) of the magnetic resonance imaging apparatus (201),
   - Determining a corrected magnetic field evolution (c) with enhanced signal-to-noise ratio based on a spatial-temporal correlation of the samples (S11, S21, S12', S22'; S11, S21, S12, S22),
   - Providing the corrected magnetic field evolution (c) for reconstructing an image or a gradient system calibration,
   - Optionally reconstructing the image or generating the gradient system calibration based on the corrected magnetic field evolution and providing an output comprising the reconstructed image or the gradient calibration.

2. Computer implemented method according to claim 1, wherein the first point in time is in a time interval (I1) including a first dynamic (D1, D1') comprising a plurality of samples (S11, S12) of the field probe array (101) and the second point is in time in a time interval (I1; I2) including a second dynamic (D2, D2') comprising a plurality of samples (S21, S22; S12'; S22') different from the first dynamic (D1, D1').

3. Computer implemented method according to claim 1, wherein the first point in time is in a dynamic (D1, D1'; D2, D2') comprising a plurality of samples (S11, S12; S21, S22) of the field probe array (101) and the second point in time is in the same dynamic (D1, D1'; D2, D2').

4. Computer implemented method according to one of the preceding claims comprising providing at least a first dynamic (D1, D1') comprising a plurality of samples (S11, S12) and a second dynamic (D2, D2') comprising a plurality of samples (S12'; S21; S22') acquired over different time intervals (I1, I2), wherein the method comprises synchronizing the predetermined time intervals (I1, I2) of acquiring the dynamics (D1, D1'; D2, D2') with an operation of the magnetic resonance imaging apparatus, in particular such that at least one parameter defining or contributing to a magnetic field generation is synchronized with the time intervals (I1, I2) of acquiring the dynamics (D1, D1'; D2, D2').

5. Computer implemented method according to one of the preceding claims, wherein the samples (S11, S12; S21; S22') were acquired at at least 3 different spatial locations (X1, X2), in particular at at least 4 different spatial locations (X1, X2), preferably at at least 16 different spatial locations (X1, X2).

6. Computer implemented method according to one of the preceding claims, wherein determining the corrected magnetic field evolution (c) includes applying a dimensionality reduction algorithm (2) to the samples (S11, S12; S11, S21; S11, S12'; S11, S22') for achieving a noise reduction, in particular (i) a PCA, SVD, or SSA -algorithm and/or (ii) a dimensionality reduction algorithm selected from at least one of a locally linear embedding, a singular spectrum analysis, a Kernel-PCA, a Kernel independent component analysis, a wavelet-based PCA, a wavelet-based independent PCA, isometric mapping, t-distributed stochastic neighbour embedding, uniform manifold approximation and projection, autoencoders, a machine learning algorithm, proper orthogonal decomposition, dynamic mode decomposition, spatial-temporal autoencoders, spatial-temporal convolutional neural networks, spatial-temporal graph neural networks, non-linear independent component analysis, spatial-temporal variational autoencoders, and/or spatial-temporal clustering.

7. Computer implemented method according to claim 6, wherein the dimensionality reduction by the PCA, SVD, or SSA algorithm (2) is chosen such as to enable a noise reduction of the corrected magnetic field evolution (c), in particular such that a noise reduced field evolution is calculated by specific weighting of singular values, in particular such that only principal components greater than or equal to a threshold (T) are selected, in particular a threshold (T) dependent on the samples (S11, S12; S21; S12'; S22') or a predefined threshold are selected.

8. Computer implemented method according to one of the preceding claims, comprising deriving the spatial-temporal correlation based on measured or derived magnetic resonance imaging apparatus properties, in particular (i) a linear transfer function and/or a non-linear transfer function of the magnetic field, (ii) derived system properties of a shim system, and/or (iii) at least one nominal sequence information, selected from a nominal gradient timing, a nominal repetition time, an echo time, and a nominal bandwidth.

9. Computer implemented method according to one of the preceding claims, wherein determining the corrected magnetic field evolution (c) includes (i) applying a noise reduction filter to the magnetic field evolution (c) or to the samples (S11, S12; S21; S12'; S22') in the spatial-temporal domain, wherein the filter is preferably selected from at least one of a Wiener filter, a Kalman filter, or a convolutional neural network filter, and a Savitzky-Golay filter, or (ii) determining the corrected magnetic field evolution (c) based on the spatial-temporal correlation is carried out by a supervised or unsupervised machine learning model, in particular a neural network, preferably a convolutional neural network.

10. A computer program product which, when the program is executed by a computer, causes the computer to carry out the steps of the computer implemented method of one of the preceding claims.

11. A computer-readable storage medium comprising the corrected magnetic field evolution (c) with enhanced signal-to-noise ratio based on a spatial-temporal correlation obtainable by the computer implemented method according to one of the claims 1 - 9.

12. A system comprising the field probe array (101), in particular a magnetic resonance imaging apparatus (201), and a processing unit (3) configured for carrying out the computer implemented method according to one of the claims 1 - 9.

13. The system according to claim 12, wherein the field probes (21, 22) of the field probe array (101) can be NMR or EPR field probes comprising an active medium selected from at least one of an aqueous solution and at least one lipophilic component, in particular at least in proximity of means for NMR or EPR excitation and/or detection, wherein a smallest dimension of an NMR/EPR active region of the active medium is preferably selected from one of the following ranges: 0.2 mm - 0.7 mm, 0.7 mm - 1.1 mm, and 1.1 mm - 2 mm.

14. Computer implemented method for enhancement of the signal-to-noise ratio of a magnetic field evolution for a magnetic resonance imaging apparatus (201), in particular according to one of the claims 1 - 9, comprising the steps of:

- Providing a first dynamic (D1) comprising a plurality of first samples (S11, S12) of the magnetic field (M), in particular comprising or consisting of gradient of the magnetic field, which are different from each other,
- Providing a second dynamic (D2) comprising a plurality of second samples (S11', S12') of the magnetic field which are different from each other, said samples (S11, S12, S11', S12') being acquired by a field probe array (101) while the field probe array (101) is placed in a magnetic field of an imaging volume (V) of the magnetic resonance imaging apparatus (201), wherein the first dynamic (D1) and the second dynamic (D2) are substantially different from each other, and
- Determining a corrected magnetic field evolution (c) with enhanced signal-to-noise ratio (SN) based on a temporal correlation of the dynamics (D1, D2).

15. The computer implemented method according to claim 14, wherein the temporal correlation is at least partially a temporal correlation of a plurality of samples (S11, S12; S11', S12') of the same dynamic (D1; D2).

16. The computer implemented method according to one of the claims 14 or 15, wherein the method comprises acquiring the at least one first dynamic (D1) and at least one second dynamic (D2) over predetermined time intervals (I1, I2), in particular the same predetermined time intervals (I1) or different time intervals (I1, I2), wherein the method comprises synchronizing the time intervals (I1, I2) of acquiring the dynamics (D1, D2) with an operation of the magnetic resonance imaging apparatus, in particular such that at least one parameter defining or contributing to a magnetic field generation is synchronized with the time intervals (I1, I2) of acquiring the dynamics (D1, D2).

Fig. 1

Fig. 2A_1

TR

I1

S11  S12

Fig. 2A_2

X1

TR

I2

S11'  S12'

D1

D2

Fig. 2A_3

Fig. 2A

S11  S12

X1

Fig. 2B_1

D1

S21  S22

X2

Fig. 2B_2

D1'

I1

Fig. 2B

I2

Fig. 2C_1

Fig. 2C_2

Fig. 2C

Fig. 2D

S11     S12                                          S12'

Fig. 2B_1                                                         X1

                    D1                              D2

Fig. 2B_2          S21                              S22'          X2

                    D1'                             D2'

                    I1          Fig. 2E             I2

$K_G(t',t)$

G1

TR    t

GTF

G2

f

$K_B(r',r,t',t)$

G3

r    t

B

G4

f

| 31 | → | 32 | → | 33 | ← | 34 |

35

36

Fig. 3

$\psi_n(t)$

t

Fig. 4A

$\mathrm{PC}_1\big(\psi_n(t)\big)$

t

Fig. 4B

Fig. 4C

Fig. 4D

Fig. 4E

$\Delta\psi_n(t)$

t

Fig. 4F

$\Delta\psi_n(t)$

t

Fig. 4G

Fig. 5A

**Standard method**

Fig. 5C

**Std-Mah method**

Fig. 5B

Fig. 5D

Fig. 5E

**Ratio**

Fig. 5F

Europäisches
Patentamt

European
Patent Office

Office européen
des brevets

## EUROPEAN SEARCH REPORT

**Application Number**

EP 24 21 7253

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WILM BERTRAM J. ET AL: "Higher order reconstruction for MRI in the presence of spatiotemporal field perturbations", MAGNETIC RESONANCE IN MEDICINE, vol. 65, no. 6, 22 April 2011 (2011-04-22), pages 1690-1701, XP093270233, US ISSN: 0740-3194, DOI: 10.1002/mrm.22767 Retrieved from the Internet: URL:https://api.wiley.com/onlinelibrary/tdm/v1/articles/10.1002%2Fmrm.22767> | 1-5,8, 10-16 | INV. G01R33/24 G01R33/44 G01R33/565 G01R33/58 ADD. G01R33/389 |
| A | * page 1690 - page 1694; figures 1,2 * | 6,7,9 | |
| X | WEZEL JOEP ET AL: "A comparison of navigators, snap-shot field monitoring, and probe-based field model training for correcting B 0 -induced artifacts in -weighted images at 7?T", MAGNETIC RESONANCE IN MEDICINE, vol. 78, no. 4, 17 November 2016 (2016-11-17), pages 1373-1382, XP093270486, US ISSN: 0740-3194, DOI: 10.1002/mrm.26524 Retrieved from the Internet: URL:https://api.wiley.com/onlinelibrary/tdm/v1/articles/10.1002%2Fmrm.26524> * page 1373 - page 1377 * | 1-16 | |

**TECHNICAL FIELDS SEARCHED (IPC)**

G01R

The present search report has been drawn up for all claims

| Place of search | Date of completion of the search | Examiner |
|---|---|---|
| Munich | 5 May 2025 | Lebar, Andrija |

EPO FORM 1503 03.82 (P04C01)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **DUBOVAN, P. I.** ; **GILBERT, K. M.** ; **BARON, C. A.** A correction algorithm for improved magnetic field monitoring with distal field probes. *Magnetic Resonance in Medicine*, 2023, vol. 90, 2242-2260